Europäisches Patentamt

**European Patent Office**

Office européen des brevets

(11) Veröffentlichungsnummer: **0 077 557**
**A2**

(12) **EUROPÄISCHE PATENTANMELDUNG**

(21) Anmeldenummer: **82109598.1**

(22) Anmeldetag: **18.10.82**

(51) Int. Cl.³: **C 12 N 15/00**
**C 12 P 35/00**
**//C12R1/645**

(30) Priorität: **21.10.81 DE 3141691**

(43) Veröffentlichungstag der Anmeldung:
**27.04.83 Patentblatt 83/17**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt/Main 80(DE)**

(72) Erfinder: **Esser, Karl, Prof., Dr.**
**Am Spik 23a**
**D-4630 Bochum(DE)**

(72) Erfinder: **Minuth, Walter**
**Brüder-Grimm-Strasse 32**
**D-6000 Frankfurt am Main 60(DE)**

(54) Plasmid pAC 1, Verfahren zu seiner Gewinnung und seine Verwendung.

(57) Plasmid pAC 1, welches aus Acremonium chrysogenum ATCC 14553 gewonnen wird, eine Konturlänge von etwa 6,7 μm und eine Molekülgröße von etwa 20,9 Kilobasen (=kb) aufweist, ein Verfahren zu seiner Gewinnung und seine Verwendung zur Herstellung eines Hybridvektors, der die Biosynthese von β-Lactam-Antibiotika fördert.

EP 0 077 557 A2

HOECHST AKTIENGESELLSCHAFT    HOE 81/F 277    0077557    Dr.KL/cr

**Plasmid pAC 1, Verfahren zu seiner Gewinnung und seine Verwendung**

Gegenstand der Erfindung ist das Plasmid pAC 1, ein Verfahren zu seiner Gewinnung aus Acremonium chrysogenum ATCC 14553 und seine Verwendung zur Herstellung eines Hybridvektors, der die Biosynthese von ß-Lactam-Antibiotika fördert.

Die Anwendung gentechnologischer Methoden auf Antibiotika produzierende Pilzstämme hat in jüngster Zeit zunehmende Bedeutung gewonnen. Hierbei soll die Aufgabe gelöst werden, genetisch modifizierte Pilzstämme zu gewinnen, die eine größere Leistungsfähigkeit bei der Bildung von Antibiotika aufweisen. Diese Aufgabe läßt sich jedoch nur dann erfolgreich mit gentechnologischen Methoden lösen, wenn ein Plasmid zur Verfügung steht, das von der genetisch zu verbessernden Wirtszelle nicht alsbald wieder eliminiert wird, wie es bei miteinander nicht verwandten Mikroorganismen häufig beobachtet wird.

Es ist bekannt, daß bestimmte Stämme verschiedener Acremonium-Arten zur Herstellung von Cephalosporinen eingesetzt werden. Da jedoch bisher weder bei Acremonium-Arten noch bei anderen nahe verwandten Pilzstämmen Plasmide gefunden werden konnten, war es noch nicht möglich, gentechnologische Methoden zur Gewinnung von Stämmen mit erhöhter Cephalosporin-Produktion einzusetzen.

Überraschenderweise wurde nun gefunden, daß in dem Stamm Acremonium chrysogenum ATCC 14553 ein Plasmid vorkommt, das eine Konturlänge von etwa 6,7 µm und eine Molekülgröße von etwa 20,9 Kilobasen (= kb) aufweist. Diesem Plasmid wurde die Bezeichnung pAC 1 gegeben.

Das Plasmid pAC 1 läßt sich durch endonukleolytische Spaltung mit Restriktionsenzymen auf Agarosegelen charakterisieren. Damit lassen sich Zahl und Größe der jeweiligen Fragmente erkennen. So spaltet die Restriktionsendonuklease Bgl II dieses Plasmid in sechs Fragmente mit den Größen 5,10, 4,75, 4,30, 3,50, 2,15 und 1,05 kb.

Dagegen zerlegt die Restriktionsendonuklease Eco R I das Plasmid pAC 1 in fünf Fragmente mit den Größen 8,1, 4,7, 4,4, 2,6 und 1,3 kb.

Die Restriktionsendonuklease Hpa I spaltet das Plasmid dagegen in neun Fragmente mit den Größen 5,61, 4,30, 3,50, 2,72, 1,35, 1,25, 0,82, 0,74 und 0,61 kb.

Dagegen konnte bisher noch keine Spaltung bei Einwirkung der Restriktionsendonukleasen Hind III, Sal I und Kpn I beobachtet werden.

Die Isolierung des Plasmid pAC 1 aus Kulturen von Acremonium chrysogenum ATCC 14553 ist vor allem deshalb kompliziert, weil in der Zelle außerdem auch noch nukleäre DNA (= nDNA) und mitochondriale DNA (=mtDNA), letztere von ähnlicher Dichte und Konturlänge, vorkommen, von denen das Plasmid pAC 1 sorgfältig abgetrennt werden muß. Es hat sich jedoch gezeigt, daß das Problem der Reindarstellung des Plasmids pAC 1 gelöst werden kann, wenn man die aus Protoplastenlysaten oder mechanisch aufgeschlossenen Myzelien von Acremonium chrysogenum ATCC 14553 gewonnene Gesamt-DNA durch Caesiumchloridzentrifugation vorreinigt, daraus die aus Plasmid-DNA und mitochondrialer DNA bestehende zirkuläre DNA chromatographisch, zum Beispiel an Hydroxylapatit,

abtrennt und die Plasmid-DNA schließlich durch mehrere, vorzugsweise drei, aufeinander folgende Caesiumchlorid-Zentrifugationen isoliert und rein darstellt. Als Ausgangsmaterial dienen Protoplasten oder nach Einfrieren in flüssigem Stickstoff mechanisch aufgeschlossene Myzelien.

Im folgenden werden bevorzugte Ausgestaltungen der Erfindung näher erläutert.

Die Freisetzung der Gesamt-DNA wird durch Inkubation mit einem Tensid wie Natriumdodecylsulfat (=SDS) und einem Protein-abbauenden Enzym wie Proteinase K erreicht; sie wird durch eine Ethanolfällung und eine Caesiumchlorid-Gradientenzentrifugation von den übrigen Zellbestandteilen gereingt. Durch eine Chromatographie an Hydroxylapatit wird bei Verwendung bestimmter Salzkonzentrationen zunächst die Gesamt-DNA spezifisch gebunden (und damit von restlichen Verunreinigungen, besonders RNA und Kohlenhydraten befreit), dann wird die niedermolekulare, zirkuläre DNA (mt- und pl-DNA) gezielt eluiert, während die nukleäre an die Säule gebunden bleibt. Die Abtrennung der hinsichtlich Struktur und Konturlänge sehr ähnlichen mitochondrialen DNA von der Plasmid-DNA erfolgt unter Ausnutzung des geringen Dichte-Unterschiedes durch einige präparative Ultra-zentrifugationsschritte in Caesiumchloridgradienten. Der Reinheitsgrad der Präparation wird durch Restriktions-analyse und elektronenmikroskopische Darstellung über-prüft.

Das Plasmid pAC 1 eignet sich vor allem zur Bildung eines Hybridvektors, der in Acremonium-Arten eingeführt werden kann. Es hat sich nämlich gezeigt, daß alle bisher bekannten Plasmide sich nur in relativ wenigen Wirtszellen etablieren lassen. Die größten Chancen zu einer erfolgreichen Klonierung bestehen immer dann, wenn der Hybridvektor in eine nahe verwandte Wirtszelle eingebracht wird. Die Verwandschaft der Cephalosporin produzierenden Stämme schafft deshalb hierfür sehr gute Voraussetzungen.

Zur Herstellung des Hybridvektors werden dabei die gleichen gentechnologischen Methoden angewendet, die bei Escherichia coli-Plasmiden bereits früher eingesetzt wurden und auch bei Streptomyceten von M. Bibb, J.C. Schottel, S.N. Cohen, Nature 284, 526-531 (1980) und C.J. Thompson, J.M Ward, D.A. Hopwood, Nature 286, 525-527 (1980) angewendet wurden.

Nach diesen bekannten Verfahren können in das Plasmid pAC 1 in einige der mit den obengenannten Restriktionsendonucleasen erhaltenen Schnittstellen nicht nur Gene, die Antibiotika-Resistenzen tragen, eingefügt werden, sondern auch Gene, die eine Steigerung der Antibiotika-Produktion bewirken. Die so erhaltenen Hybrid-Plasmide sind nach allen bisher vorliegenden Beobachtungen in Acremonium-Zellen ebenso lebens- und vermehrungsfähig wie das Ausgangsplasmid.

Die Erfindung wird durch das folgende Beispiel weiter veranschaulicht:

<u>Beispiel</u>

Ausgangsstamm für die Isolation der Plasmid-DNA ist
der Stamm Acremonium chrysogenum ATCC 14553. Das Myzel
dieses Stammes wird etwa 48 Stunden lang unter Schütteln
in einem Komplett-Flüssigmedium folgender Zusammensetzung angezogen:

| | |
|---|---|
| Saccharose | 3,0 g |
| Dextrin (weiß) | 15,0 g |
| NaCl | 0,5 g |
| $K_2HPO_4$ | 0,5 g |
| $MgSO_4 \cdot 7\ H_2O$ | 0,5 g |
| $FeSO_4 \cdot 7\ H_2O$ | 0,01 g |
| Trypticase-Soy-Broth (Difco) | 5,0 g |
| Fleischextrakt (Difco) | 1,0 g |
| Hefeextrakt (Difco) | 2,0 g |
| Agar (Serva) | 20 g |
| Dest. $H_2O$ | ad 1000 ml |

Dieses Medium war vorher 20 Minuten bei 121°C sterilisiert
worden. Der pH-Wert stellte sich hier auf 7,0 bis
7,2 ein.

Nach 48 Stunden wurde das Myzel über Mull geerntet und
unter flüssigem Stickstoff nach dem von U.Stahl et al.
/¯Molec. Gen. Genet. 178, 639 ff (1980)_7 beschriebenen
Verfahren aufgeschlossen. Für das weitere Verfahren
werden etwa 30 g Myzel eingesetzt.

Der Zellaufschluß kann aber auch mit Hilfe von Enzymen
durchgeführt werden. Dazu wird das von der Kulturlösung abgetrennte Myzel zweimal mit destilliertem
Wasser gewaschen, in einer isotonischen, neutralen,
0,5 Gew.-% 2-Mercaptoethanol enthaltenden Lösung
suspendiert und bei 30°C 30 Minuten stationär inkubiert.
Danach wird das Myzel mit einer  isotonischen,neutralen Lösung gewaschen. Auf dieses Myzel läßt man dann 12 mg/ml des Cytophaga-Enzyms $L_1$ und 2 mg/ml Zymolase in einer isotonischen, neutralen Lösung bei leichtem Schütteln und einer Temperatur von 30°C etwa 3 Stunden lang einwirken. Die Protoplasten werden dann durch eine Filtration (Glaswattefilter) von den restlichen Myzelstücken getrennt und zweimal durch eine fünfminütige Zentrifugation in isotonischer Lösung gewaschen.

Zu der Protoplastensuspension wird dann Ethylendiamintetraacetat als Nukleasehemmer bis zu einer Endkonzentration von 60 mM gegeben. Dann wird zur Freisetzung der Gesamt-DNA zunächst Natriumdodecylsulfat bis zu einer Endkonzentration von 1 % und Proteinase K (Merck) bis zu einer Endkonzentration von 0,02 Gew.-% zugegeben, dann 30 Minuten bei 37°C stehengelassen und schließlich 15 Minuten bei 70°C inkubiert. Das Rohlysat wird durch Zentrifugation (10 min bei 10 000 g und 4°C) geklärt und dann 15 Stunden gegen einen Puffer bestehend aus 50 mM Trishydroxymethylaminomethan und 20 mM Ethylendiamintetraacetat dialysiert. Die Gesamt-DNA wird durch Zugabe von 2 Volumen Ethanol ausgefällt und nach 15 stündigem Stehen bei -20°C abzentrifugiert (8 Minuten mit 3000 g bei -20°C) und durch isopyknische Gradientenzentrifugation in einer Caesiumchlorid-Lösung, die auf die Dichte 1,700 eingestellt ist, vorgereinigt (21 Stunden mit 45 000 Upm bei 15°C im Sorvall-Rotor TV 865). Die DNA-Banden werden mit Hilfe eines Isco-Fraktionators gewonnen.

Zur Abtrennung der zirculären DNA, die aus Plasmid-DNA und mitochondrialer DNA besteht, von der Gesamt-DNA wird das Gemisch gegen einen Puffer dialysiert, der aus 50 mM Trishydroxymethylaminomethan, 20 mM Ethylendiamintetraacetat und 240 mM Natriumdihydrogenphosphat besteht und einen pH 6,7 aufweist. Das Dialysat wird an Hydroxylapatit nach dem von Colman et al. beschriebenen

Verfahren chromatographiert. (Eur.J. Biochem. 91, 303 ff (1978)). Bei diesem Verfahren wird zunächst die Gesamt-DNA an einer Säule von 2 g Hydroxylapatit gebunden, anschließend die zirkuläre DNA mit Pufferlösungen ausgewaschen, während die nukleäre DNA am Apatit gebunden bleibt.

Die Abtrennung der mitochondrialen DNA und damit die Reindarstellung der Plasmid-DNA erfolgt durch drei aufeinanderfolgende isopyknische Gradientenzentrifugationen in einer Caesiumchloridlösung, die auf eine Dichte von 1,700 eingestellt ist (44 Stunden mit 32 000 Upm bei 15°C im Sorvall-Rotor TV 865).

## PATENTANSPRÜCHE

1. Plasmid pAC 1, dadurch gekennzeichnet, daß es aus Acremonium chrysogenum ATCC 14553 erhältlich ist, eine Konturlänge von etwa 6,7 µm und eine Molekülgröße von etwa 20,9 Kilobasen (= kb) aufweist.

2. Plasmid nach Anspruch 1, dadurch gekennzeichnet, daß es von der Restriktionsendonuklease Bgl II in sechs Fragmente mit den Größen 5,10, 4,75, 4,30, 3,50, 2,15 und 1,05 kb zerlegt wird.

3. Plasmid nach Anspruch 1, dadurch gekennzeichnet, daß es von der Restriktionsendonuklease Eco R I in fünf Fragmente mit den Größen 8,1, 4,7, 4,4, 2,6 und 1,3 kb zerlegt wird.

4. Plasmid nach Anspruch 1, dadurch gekennzeichnet, daß es von der Restriktionsendonuklease Hpa I in neun Fragmente mit den Größen 5,61, 4,30, 3,50, 2,72, 1,35, 1,25, 0,82, 0,74 und 0,61 kb zerlegt wird.

5. Verfahren zur Gewinnung des Plasmids pAC 1, dadurch gekennzeichnet, daß man die aus Protoplastenlysaten oder mechanisch aufgeschlossenen Myzelien von Acremonium chrysogenum ATCC 14553 gewonnene Gesamt-DNA durch Caesiumchlorid-Zentrifugation vorreinigt, daraus die aus Plasmid-DNA und mitochondrialer DNA bestehende zirkuläre DNA chromatographisch abtrennt und die Plasmid-DNA schließlich durch mehrere aufeinander folgende Caesiumchlorid-Zentrifugationen isoliert und rein darstellt.

6. Verfahren nach Anspruch 5, dadurch gekennzeichnet, daß die zirkuläre DNA von der nucleären DNA durch Chromatographie an Hydroxylapatit abgetrennt wird.

7.  Verwendung des Plasmids nach den Ansprüchen 1-4
    zur Herstellung eines Hybridvektors, der die Biosynthese von ß-Lactam-Antibiotika fördert.

Patentansprüche für Österreich

1. Verfahren zur Gewinnung des Plasmids pAC 1, das eine Konturlänge von etwa 6,7 µm und eine Molekülgröße von etwa 20,9 Kilobasen (= kb) aufweist, dadurch gekennzeichnet, daß man die aus Protoplastenlysaten oder mechanisch aufgeschlossenen Myzelien von Acremonium chrysogenum ATCC 14553 gewonnene Gesamt-DNA durch Caesiumchlorid-Zentrifugation vorreinigt, daraus die aus Plasmid-DNA und mitochondrialer DNA bestehende zirkuläre DNA chromatographisch abtrennt und die Plasmid-DNA schließlich durch mehrere aufeinander folgende Caesiumchlorid-Zentrifugationen isoliert und rein darstellt.

2. Verfahren nach Anspruch 1, dadurch gekennzeichnet, daß die zirkuläre DNA von der nucleären DNA durch Chromatographie an Hydroxylapatit abgetrennt wird.

3. Verwendung des nach Anspruch 1 erhaltenen Plasmids zur Herstellung eines Hybridvektors, der die Biosynthese von ß-Lactam-Antibiotika fördert.